(19) Europäisches Patentamt
European Patent Office
Office européen des brevets

(11) EP 1 215 271 B1

# (12) FASCICULE DE BREVET EUROPEEN

(45) Date de publication et mention de la délivrance du brevet:
**06.05.2004 Bulletin 2004/19**

(51) Int Cl.[7]: **C09K 19/58**, C07D 493/04

(21) Numéro de dépôt: **00204584.7**

(22) Date de dépôt: **18.12.2000**

## (54) Dopants chiraux à induction de pas d'hélice variable et leur application à un affichage réflectif en couleur

Chirale Dotierstoffe zur Erzeugung einer variablen Helixganghöhe und deren Anwendung in einer mehrfarbigen reflektierenden Anzeige

Chiral dopants for induction of a variable helical pitch and their application in a full colour reflective display

(84) Etats contractants désignés:
**AT BE CH CY DE DK ES FI FR GB GR IE IT LI LU MC NL PT SE TR**

(43) Date de publication de la demande:
**19.06.2002 Bulletin 2002/25**

(73) Titulaire: **ASULAB S.A.**
**2074 Marin (CH)**

(72) Inventeurs:
- **Chuard, Thierry**
  **2206 Les Geneveys-sur-Coffrane (CH)**
- **Deschenaux, Robert**
  **2300 La Chaux-de-Fonds (CH)**
- **Klappert, Rolf**
  **2000 Neuchâtel (CH)**
- **Meyer, Séverine**
  **2000 Neuchatel (CH)**

(74) Mandataire: **Thérond, Gérard Raymond et al**
**I C B**
**Ingénieurs Conseils en Brevets SA**
**Rue des Sors 7**
**2074 Marin (CH)**

(56) Documents cités:
**EP-A- 0 755 915**
**WO-A-98/34995**
**WO-A-98/55473**
**DE-A- 19 520 704**
**DE-A- 19 738 642**
**DE-A- 19 859 584**

- **WITTE VAN DE P ET AL: "MODIFICATION OF THE PITCH OF CHIRAL NEMATIC LIQUID CRYSTALS BY MEANS OF PHOTOISOMERIZATION OF CHIRAL DOPANTS" LIQUID CRYSTALS,GB,TAYLOR AND FRANCIS LTD, LONDON, vol. 24, no. 6, 1 juin 1998 (1998-06-01), pages 819-827, XP000773004 ISSN: 0267-8292**
- **YARMOLENKO S N ET AL: "PHOTOSENSITIVE CHIRAL DOPANTS WITH HIGH TWISTING POWER" LIQUID CRYSTALS,GB,TAYLOR AND FRANCIS LTD, LONDON, vol. 16, no. 5, 1 mai 1994 (1994-05-01), pages 877-882, XP000442136 ISSN: 0267-8292**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 215 271 B1

## Description

La présente invention concerne une nouvelle famille de dopants chiraux à induction de pas d'hélice variable, c'est-à-dire des composés chimiques qui permettent, lorsqu'ils sont ajoutés en petites quantités à une composition cristal liquide de type nématique d'induire un pas d'hélice variable en fonction d'une isomérisation d'une partie de la structure chimique, par exemple au moyen d'une irradiation appropriée dont la longueur d'onde peut aller de l'ultraviolet (UV) au visible (VIS), ou au moyen d'un gradient thermique.

L'invention concerne plus particulièrement de tels dopants chiraux dans lesquels les chaînes terminales comportent un reste polymérisable permettant de fixer le pas d'hélice induit par isomérisation, et donc de fixer une ou plusieurs couleurs déterminées en utilisant une seule composition cristal liquide à laquelle est ajouté un unique dopant chiral selon l'invention ou une unique composition desdits dopants chiraux.

Dans les afficheurs en mode réflectif utilisant des matériaux à phase nématique chirale, également désignée par phase cholestérique, il est connu de fixer une couleur déterminée par l'utilisation:

- d'une substance pure optiquement active présentant une phase nématique chirale,
- d'un mélange de substances optiquement actives présentant toutes une phase nématique chirale, ou
- d'une substance ou d'un mélange de substances achirales présentant une phase nématique et contenant une ou plusieurs substances optiquement actives, c'est-à-dire des dopants chiraux pouvant être mésomorphes ou non et capables d'induire une hélicité ayant un pas déterminé dans toute la mésophase pour former une phase cholestérique.

Les composés selon la présente invention correspondent aux dopants chiraux de la dernière catégorie et appartiennent plus généralement à la catégorie des commutateurs moléculaires photochimiques, dont certaines familles ont déjà fait l'objet de nombreuses études et publications qui sont citées ci-après à titre illustratif mais non exhaustif.

La photoracémisation du binaphtyle et de ses dérivés, ainsi que leur capacité d'induction d'hélicité dans le milieu nématique ont fait l'objet d'études approfondies en particulier par H.J. Deussen & al (Liq. Cryst. 1996, 21, 327; Mat. Res. Soc. Symp. Proc. 1996, 425, 55). Il apparaît toutefois que la photoisomérisation de ces composés entraîne une décomposition importante défavorable à la persistance d'une couleur déterminée.

B. L. Feringa & al (J. Am. Chem. Soc. 1991, 113, 5468) ont étudié des composés de type thioxanthène qui isomérisent réversiblement entre deux formes diastéréoisomériques, avec l'objectif d'appliquer ce système au stockage réversible d'informations et permettant donc également l'application à un affichage en couleur. Ces composés ont toutefois l'inconvénient de présenter une stabilité chimique insuffisante et une vitesse de commutation beaucoup trop longue (plusieurs minutes, voire des heures), incompatible par exemple avec un affichage digital dans une pièce d'horlogerie. Dans le même domaine, l'équipe de B. L. Feringa a également étudié des dérivés de type dithiényléthène photoisomérisables entre une forme ouverte et une forme fermée (L. N. Lucas & al Chem. Commun. 1998, 2313; Tetrahedron Lett. 1999, 40, 1775).

G. B. Schuster & al (J. Am. Chem. Soc, 1995, 117, 8524) ont mis à profit les travaux de Y. Yokoyama & al (J. Am. Chem. Soc., Chem. Comm. 1995, 785) sur les dérivés du fulgide pour modifier l'hélicité d'un matériau cholestérique. Il apparaît toutefois qu'il faut une concentration en dérivés de fulgide élevée (≥ 5%), pour obtenir un changement du pas d'hélice seulement modéré (environ 30%).

H. Hattori & al (Liq. Cryst. 1999, 26, 1085; J. Polym. Sci. 2000, 38, 887) ont étudié des dérivés du spiropyrane, et plus particulièrement des composés obtenus par la copolymérisation d'un dérivé du cholestérol avec différents monomères contenant une unité spiropyrane fonctionnalisée sur d'autres positions que sur l'azote. Il apparaît toutefois que la modification du HTP par exposition à l'UV est insuffisante et que la modification de la couleur réfléchie par ce matériau ($\Delta\lambda$ environ 10 nm) est insuffisante pour couvrir le spectre visible et envisager la réalisation d'un affichage trichrome.

On peut également signaler les travaux de S.N. Yarmolenko & al (Liq. Cryst. 1994, 16, 877) sur les unités chirales photomodulables dérivées du menthyle et les travaux de R. P. Lemieux & al (Liq. Cryst. 1996, 20, 741; J. Am. Chem. Soc. 1997, 119, 8111 ) sur les unités chirales photomodulables dérivées du thioindigo.

Les photocommutateurs moléculaires chiraux, dont les caractéristiques ont brièvement été rappelées ci-dessus intègrent pratiquement tous l'unité chirale et l'unité photoactive dans une même entité.

Les dopants chiraux selon la présente invention comprennent au contraire une unité chirale bifonctionnelle, dont au moins une des fonctions permet d'établir une liaison chimique avec un groupe isomérisable et permet donc d'avoir un groupe isomérisable séparé de l'unité chirale. Dans un mode de réalisation préféré le groupe isomérisable possède un enchaînement terminal polymérisable ou copolymérisable

Ainsi les dopants chiraux selon l'invention possèdent trois caractéristiques par irradiation UV ou VIS et/ou par adjonction de photoinitiateurs. structurales essentielles:

- une unité chirale centrale bifonctionnalisée permettant l'induction d'hélicité dans la phase nématique,
- au moins une unité isomérisable permettant de faire varier la structure moléculaire et donc de moduler l'hélicité dans la phase nématique, et
- des fonctions polymérisables permettant, d'une part en quelque sorte d'attacher les molécules par chaque extrémité et de bloquer la réaction d'isomérisation à un pas d'hélice déterminé, donc à une couleur déterminée, d'autre part, par la formation d'un gel, d'éviter les phénomènes de diffusion d'un pixel à l'autre lorsqu'on souhaite réaliser un affichage trichrome, comme cela sera expliqué dans la description détaillée.

Dans la suite de la description, "l'unité isomérisable" sera désignée par "unité photoisomérisable" en considérant que l'isomérisation est obtenue par irradiation UV ou VIS, les dopants chiraux selon l'invention étant alors désignés par dopants chiraux photomodulables.
D'autres caractéristiques et avantages de la présente invention apparaîtront plus clairement à la lecture de la description détaillée qui suit, en référence aux dessins annexés dans lesquels :

- la figure 1 représente un schéma de synthèse d'un dopant chiral selon l'invention;
- la figure 2 représente un schéma de synthèse de quatre dopants chiraux selon l'invention à partir d'un intermédiaire de synthèse commun;
- les figures 3 et 4 sont des graphiques donnant le pourcentage de transmission en fonction de la longueur d'onde pour deux composition cristal liquide cholestérique contenant un dopant chiral selon l'invention;
- la figure 5 représente schématiquement un mode de réalisation d'une cellule trichrome, et
- les figures 6 et 7 sont des graphiques donnant le pourcentage de transmission en fonction de la longueur d'onde pour deux modes de réalisation d'une cellule trichrome.

Les dopants chiraux photomodulables selon la présente invention répondent plus précisément à la formule I suivante:

$$A_1\text{-}Y_1\text{-}X\text{-}Y_2\text{-}A_2 \qquad (I)$$

dans laquelle

- X représente un radical chiral dérivant d'un composé bifonctionnalisé, tels que les diacides ou les diols,
- $Y_1$ et $Y_2$ sont identiques ou différents et représentent chacun une unité de liaison fonctionnelle choisie parmi -O-, -S-, -COO-, -OCO-, -CON(R)-, -N(R)CO- dans laquelle R représente un reste alkyle,
- $A_1$ représente un groupe photoisomérisable correspondant à la formule (II)

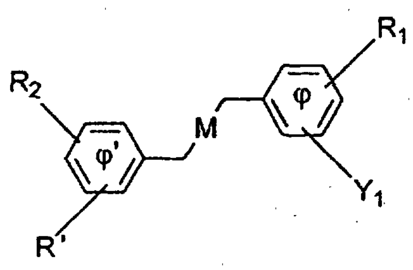

(II)

dans laquelle deux restes phenyle φ et φ' sont liés par un radical bivalent photoisomérisable -M- choisi parmi -N=N-, -N=CH-, -CH=N-, et -CH=CH-; lesdits restes phényle ayant respectivement un ou plusieurs substituants $R_1$, $R_2$ identiques ou différents choisis parmi l'hydrogène, les radicaux alkyle ou alkoxy de $C_1$ à $C_5$, les halogènes et les radicaux cyano, nitro, ou trifluorométhyle; et R' représente l'hydrogène ou un groupe correspondant à la formule (III)

$$-Y_3\text{-}C_nH_{2n}\text{-}Z \qquad (III)$$

dans laquelle $Y_3$ a les mêmes significations que $Y_1$ et $Y_2$ ou représente le phénylène, n peut prendre les valeurs de 0 à 12 et Z représente l'hydrogène ou un reste polymérisable choisi parmi les restes acryloyle et acryloyloxy; et

-$A_2$ a la même signification que les groupes de formule (II) ou de formule (III).

Parmi les diacides et les diols chiraux, conduisant au radical chiral X de la formule (I), on peut citer l'acide di-*O*,*O*'-*p*-toluyl tartrique, l'acide di-*O*,*O*'-*p*-pivaloyl tartrique, l'acide cyclohexane 1,2-dicarboxylique, l'acide camphrique, l'acide isopropylidène tartrique, l'acide 3-méthyladipique, ainsi que le dianhydro-glucitol et le dianhydro-mannitol, ces deux diols permettant la synthèse des composés préférés comme on le verra par la suite.

L'unité photoisomérisable représenté par $A_1$ et/ou $A_2$ dans la formule (I) a pour structure de base l'azophényle lorsque M représente -N=N-, l'azomethine lorsque M représente -N=CH- et le stilbène lorsque M représente -CH=CH-. Comme on verra par la suite la structure de base correspondant à l'azophényle a été retenue à titre illustratif car il s'agit d'une structure simple, facilement synthétisable et dont l'équilibre *trans* $\leftrightarrows$ *cis* est bien connu, avec un déplacement vers la forme *cis* par irradiation à une longueur d'onde $\lambda$ < 400 nm, et un déplacement vers la forme *trans* thermiquement ou par irradiation à une longueur d'onde $\lambda$ > 400 nm.

Un composé répondant à la formule générale I, et dont les caractéristiques et propriétés seront décrites plus loin, est par exemple le composé référencé 209s et répondant à la formule ci-après

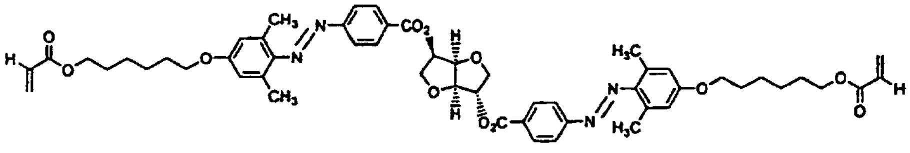

**209s**

dans laquelle le radical chiral X dérive du 1,4;3,6-dianhydro-*D*-glucitol, $Y_1$ et $Y_2$ représentent chacun -COO-, $A_1$ et $A_2$ sont identiques et représentent chacun un radical azophényle (-M- = -N=N-), dont un reste phényle ($\varphi$) non substitué ($R_1$=H) est lié en para à -COO-, et dont l'autre reste phényle ($\varphi$') disubstitué en 2',5' par un radical méthyle ($R_2$=$CH_3$) comporte en outre en para une chaîne comprenant successivement -O- (=$Y_3$), le radical hexaméthylène (n=6) et un reste polymérisable acryloyloxy (=Z).

Les structures chimiques d'autres composés illustrant la famille de dopants chiraux selon l'invention sont rapportées dans le tableau 1 ci-après avec leur caractérisation physico-chimique (point de fusion/enthalpie; analyse élémentaire).

Tableau 1

| | Structure | F °C (ΔH k/mol) | | |
|---|---|---|---|---|
| Référence | | % | Calc. | Trouv. |
| **201b** | 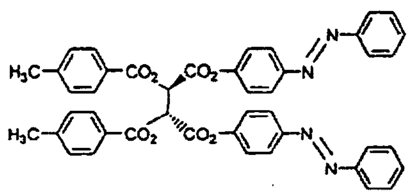 | 213 (69) | | |
| | | C | 70,77 | 70,73 |
| | | H | 4,59 | 4,67 |
| | | N | 7,50 | 7,57 |
| **201c** | 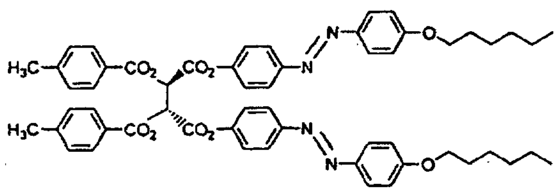 | 159 (67) | | |
| | | C | 71,02 | 70,82 |
| | | H | 6,17 | 6,15 |
| | | N | 5,92 | 5,84 |
| **201e** | 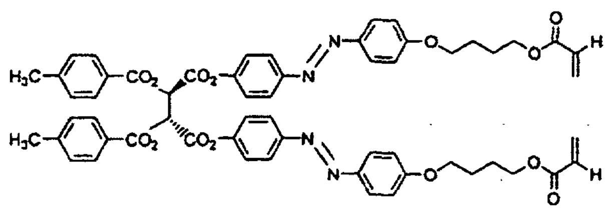 | 134 (40) | | |
| | | C | 67,56 | 67,50 |
| | | H | 5,28 | 5,27 |
| | | N | 5,43 | 5,41 |
| **202c** | 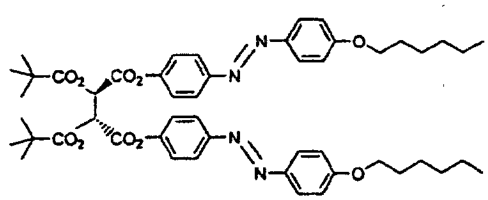 | 149 (44) | | |
| | | C | 68,32 | 68,33 |
| | | H | 7,11 | 7,10 |
| | | N | 6,37 | 6,34 |
| **203b** | 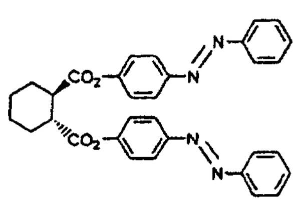 | 165 (58) | | |
| | | C | 72,17 | 71,99 |
| | | H | 5,30 | 5,40 |
| | | N | 10,52 | 10,28 |

Tableau 1 (suite)

| Référence | Structure | F°C (ΔH k/mol) | | |
|---|---|---|---|---|
| | | % | Calc. | Trouv |
| **203c** | 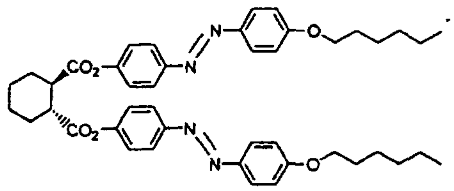 | 195 (64) | | |
| | | C | 72,11 | 72,02 |
| | | H | 7,15 | 7,10 |
| | | N | 7,64 | 7,71 |
| **204c** | 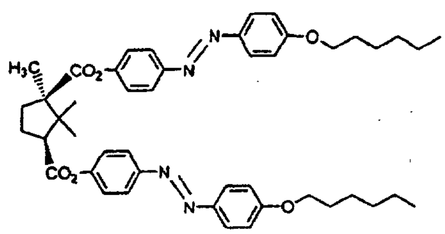 | 150 (46) | | |
| | | C | 72,61 | 72,56 |
| | | H | 7,42 | 7,40 |
| | | N | 7,36 | 7,31 |
| **206c** | 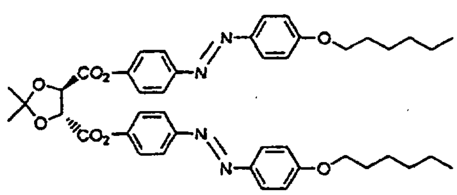 | 133 (57) | | |
| | | C | 68,78 | 68,77 |
| | | H | 6,71 | 6,74 |
| | | N | 7,46 | 7,36 |
| **306c** | 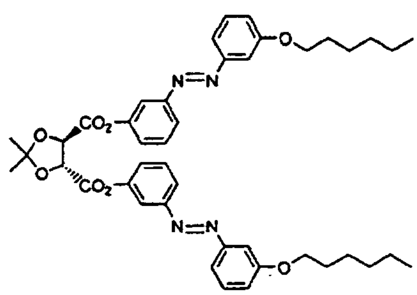 | 63 (41) | | |
| | | C | 68,78 | 68,82 |
| | | H | 6,71 | 6,82 |
| | | N | 7,46 | 7,20 |
| **206e** | 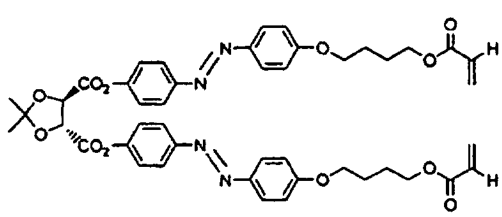 | 121 (41) | | |
| | | C | 64,67 | 64,58 |
| | | H | 5,55 | 5,69 |
| | | N | 6,71 | 6,65 |

Tableau 1 (suite)

| Structure<br>Référence | F°C (ΔH k/mol) | | |
|---|---|---|---|
| | % | Calc. | Trouv |
| **207c** | 151 (35) | | |
| 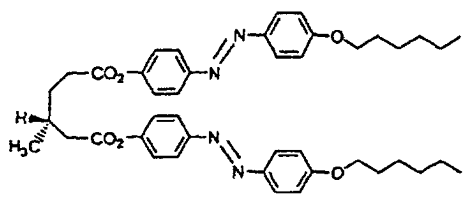 | C | 71,64 | 71,57 |
| | H | 7,27 | 7,53 |
| | N | 7,77 | 7,66 |
| **209g** | 204 (62) | | |
| 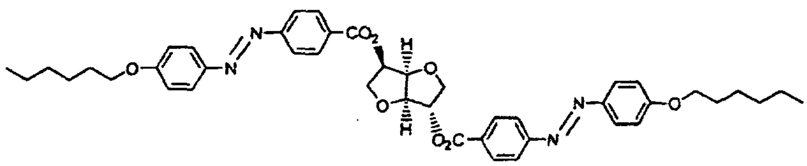 | C | 69,27 | 69,22 |
| | H | 6,61 | 6,87 |
| | N | 7,34 | 7,47 |
| **209j** | 169 (58) | | |
| 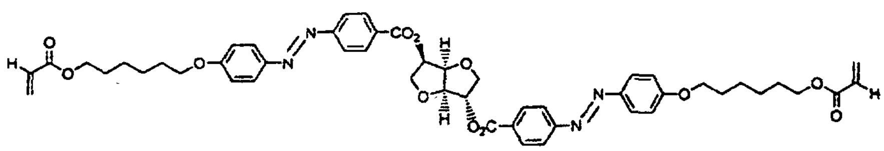 | C | 66,51 | 66,25 |
| | H | 6,03 | 6,13 |
| | N | 6,20 | 6,39 |
| **209s** | 93 (66) | | |
| 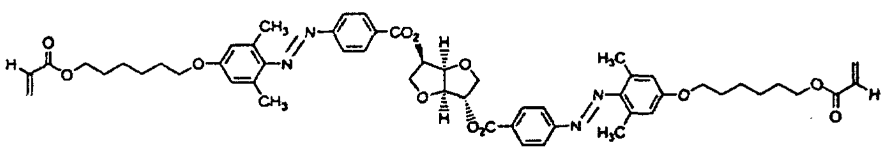 | C | 67,63 | 67,61 |
| | H | 6,52 | 6,56 |
| | N | 5,84 | 5,91 |
| **209sf** | 90 (44) | | |
| 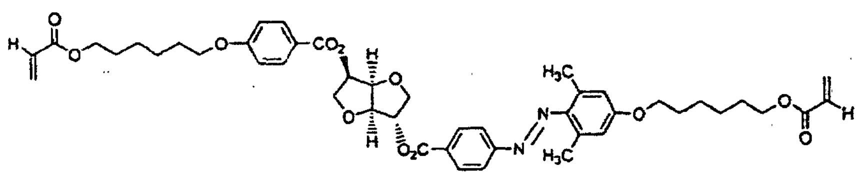 | C | 66,81 | 66,80 |
| | H | 6,58 | 6,51 |
| | N | 3,39 | 3,34 |

Tableau 1 (suite)

| Structure<br>Référence | F °C (ΔH k/mol)<br>% | Calc. | Trouv |
|---|---|---|---|
| **209fs** | 89 (46) | | |
| 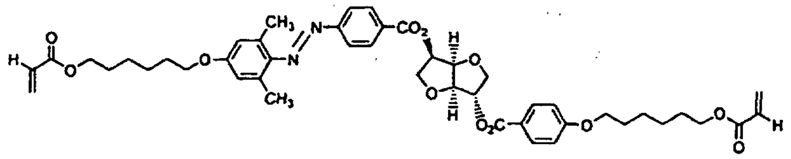 | C | 66,81 | 66,78 |
| | H | 6,58 | 6,77 |
| | N | 3,39 | 3,49 |
| **209sa** | 53 (45) | | |
| 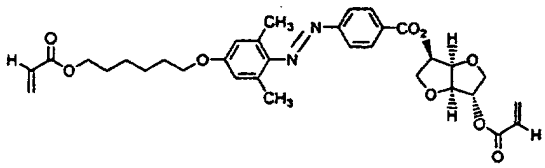 | C | 65,33 | 65,27 |
| | H | 6,31 | 6,48 |
| | N | 4,62 | 4,58 |
| **210fs** | 68 (48) | | |
| 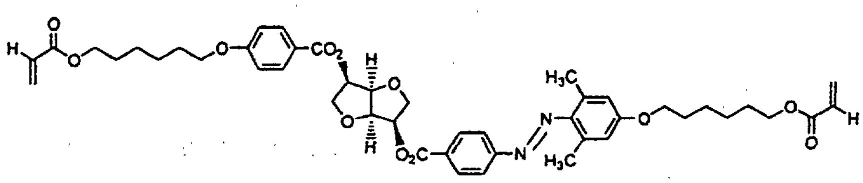 | C | 66,81 | 66,77 |
| | H | 6,58 | 6,78 |
| | N | 3,39 | 3,47 |

En se référant maintenant au schéma de synthèse représenté en annexe à la figure 1, on décrit ci-après dans l'exemple 1 la synthèse d'un composé photoisomérisable et polymérisable ayant la référence **201e** dans le tableau 1.

Exemple 1: Synthèse du 2,3-Bis[di-4,4'(4-acryloyloxybutyloxy)azophénylcarbonyloyl]-di-*O*,*O*'-*p*-toluyl-*L*-tartrate (Ref **201e**)

La synthèse de ce composé s'effectue en trois étapes.

1ère étape: Préparation de l'intermédiaire 4,4'-hydroxy-azobenzène (**200a**)

10,0 g (71,9 mmol) de 4-nitrophénol sont broyés finement dans un mortier avec 50,0 g (891,0 mmol) de KOH. Le mélange est placé dans une coupelle en porcelaine, 10 ml d'eau sont ajoutés et le tout est chauffé doucement sous agitation jusqu'à l'obtention d'une pâte homogène. Le chauffage est alors augmenté et le mélange passe progressivement du jaune au noir violacé dans une réaction vigoureuse avec dégagement gazeux. Lorsque le dégagement gazeux est terminé, le chauffage est arrêté. Le solide est dissous encore chaud dans le minimum d'eau (env. 150 ml) et le pH de la solution ainsi obtenue est amené entre 8 et 9 sous forte agitation avec HCl 5N. Un changement de couleur du brun foncé au beige a lieu ainsi que la formation d'un précipité qui est filtré sur büchner et séché à l'étuve à vide en présence de $P_2O_5$, pour donner 5,8 g de produit brut. Celui-ci est purifié par chromatographie sur gel de silice avec de l'éther comme éluant. La fraction contenant le produit pur est évaporée à sec, donnant 4,62 g de produit correspondant à la référence **200a** (rendement 60%).

2ème étape: Préparation de l'intermédiaire 4-hydroxy-4'(4-acryloyloxybutyloxy)-azobenzène (**200e**)

0,80 g (3,73 mmol) du composé **200a** et 0,54 g (3,73 mmol) de 4-hydroxybutylacrylate sont dissous dans 20 ml de THF sec. 1,00 g (3,80 mmol) de triphénylphosphine sont ajoutés et la solution est refroidie à 0 °C. 1,70 ml (3,74

mmol) d'une solution à 40 % de DEAD (diéthylazodicarboxylate) dans le toluène sont ajoutés goutte à goutte puis on laisse réagir une nuit à température ambiante. Le solvant est éliminé à l'évaporateur rotatif pour donner 3,2 g de produit brut qui est purifié par chromatographie sur gel de silice avec un éluant $CH_2Cl_2$/AcOEt (97:3). Une des fractions collectées permet d'obtenir, après élimination du solvant, 0,48 g de produit correspondant à la référence **200e** (rendement 36%)

3$^{ème}$ étape: Obtention du composé du titre

0,49 g (1,28 mmol) d'acide (-)-di-*O*,*O*'-*p*-toluyl-*L*-tartrique, 0,55 g (2,67 mmol) de DCC (N,N'-dicyclohexylcarbodiimide) et 0,80 g (2,68 mmol) du composé **200e** sont dissous dans 70 ml de $CH_2Cl_2$ sec et refroidis à 0 °C. 0,04 g (0,33 mmol) de 4-DMAP (4-diméthylamino pyridine) sont ajoutés et le mélange réactionnel est agité sept heures à 0 °C avant d'être lavé avec HCl 0,01 N. La phase organique est séchée sur $MgSO_4$ et le solvant éliminé à l'évaporateur rotatif, pour donner 1,62 g de produit brut. Celui-ci est purifié par chromatographie sur gel de silice avec au début un éluant $CH_2Cl_2$/AcOEt (98:2) puis $CH_2Cl_2$/AcOEt (94:6). 0,76 g de produit sont isolés et recristallisés à chaud dans l'EtOH, pour donner 0,40 g de produit du titre (rendement 33 %).

Outre les caractéristiques rapportées dans le tableau 1, on a également effectué le spectre de résonance magnétique nucléaire et le spectre de masse qui confirment la structure et la pureté du composé attendu:
$^1$H-RMN (200 MHz, $CDCl_3$): 1.82-1.98 (*m*, 8H, aliph.); 2.45 (*s*, 6H, $CH_3$); 4.09 (*t*, 4H, $OCH_2$); 4.26 (*t*, 4H, $CO_2CH_2$); 5.82 (*dd*, 2H, vinyl.); 6.13 (*dd*, 2H, vinyl.); 6.40 (*s*, 2H, CH); 6.42 (*dd*, 2H, vinyl.); 6.99 (*d*, 4H, arom.); 7.19 (*d*, 4H, arom.); 7.31 (*d*, 4H, arom.); 7.85 (*d*, 4H, arom.); 7.88 (*d*, 4H, arom.); 8.10 (*d*, 4H, arom.).
MS : 1031 $(M+H)^{+\cdot}$.

En se référant maintenant au schéma de synthèse représenté en annexe à la figure 2, on décrit dans les exemples 2 à 5 ci-après la synthèse des composés ayant les références **209s, 209sf, 209fs** et **209sa** dans le tableau 1.

Exemple 2: Synthèse du 2,5-Bis [4'-(6-acryloyloxyhexyloxy)-2',5'-méthylazophényl-4-carbonyloyl]-1,4;3,6-dianhydro-*D*-glucitol (**209s**).

Cette synthèse est effectuée en cinq étapes car il est nécessaire de préparer une forme réactive de l'azobenzène substitué, qui n'est pas un produit commercial.

1ère étape: Préparation de l'intermédiaire 2,5-diméthyl-4-hydroxy-4'-méthoxycarbonyl-azobenzène (**200p**)

5,00 g (33,1 mmol) de 4-aminobenzoate de méthyle sont dissous dans 40 ml d'HCl 3N et refroidis à 0 °C. Une solution de 2,76 g (40,0 mmol) de $NaNO_2$ dans 15 ml d'eau est ajoutée goutte à goutte puis le mélange est agité une heure encore à 0 °C. 0,57 g (9,54 mmol) d'urée sont ajoutés pour détruire l'excès de $NaNO_2$. Le mélange réactionnel est ensuite versé dans une solution à 0 °C de 4,04 g (33,1 mmol) de 3,5-diméthylphénol dans 40 ml de NaOH 2N entraînant la formation d'un précipité orange très dense. Le précipité est filtré sur büchner et séché à l'étuve à vide en présence de $P_2O_5$, donnant, après recristallisation à chaud dans le toluène, 7,83 g de produit ayant la référence **200p** (rendement 83 %).

2ème étape: Préparation de l'intermédiaire 2,5-diméthyl-4-(6-hydroxyhéxyloxy) azobenzène (**200g**)

8,00 g (28,1 mmol) de **200p** sont dissous dans 160 ml d'EtOH sec et 1,13 g (28,2 mmol) de NaOH sont ajoutés. Le mélange est chauffé à reflux et 3,80 g (27,8 mmol) de 6-chloro-1-hexanol sont ajoutés. Le reflux est maintenu durant six jours en ajoutant encore deux portions de 3,80 g (27,8 mmol) de 6-chloro-1-hexanol (après un et quatre jours de réaction respectivement). Après refroidissement, le mélange est concentré à environ 100 ml à l'évaporateur rotatif et transvasé dans un entonnoir à séparation. 300 ml d'éther sont ajoutés et la phase organique est lavée avec HCl 1N. La phase aqueuse étant colorée en orange, celle-ci est extraite une fois avec de l'éther. Les phases organiques sont groupées, séchées sur $MgSO_4$ et le solvant éliminé à l'évaporateur rotatif, donnant un résidu visqueux qui est purifié par chromatographie sur silice avec un éluant $CH_2Cl_2$/AcOEt (95:5). Cette opération permet de récupérer une fraction contenant le produit de référence 200q qui est évaporée à sec pour donner 11,7 g de produit sous forme huileuse.

3ème étape: Préparation de l'intermédiaire acide 2',5'-diméthyl-4'-(6-hydroxyhexyloxy)-4-azophénylcarboxylique (**200r**)

L'huile obtenue à l'étape précédente (11,7 g) est dissoute dans 150 ml d'EtOH et 200 ml d'eau contenant 9,00 g (225 mmol) de NaOH sont ajoutés et le mélange est chauffé trois heures à reflux. Après refroidissement, le mélange

est versé dans un bain eau/glace et la solution est acidifiée avec HCl 5N. Le précipité orange est filtré sur büchner et séché à l'étuve à vide en présence de $P_2O_5$. La recristallisation à chaud dans l'AcOEt donne 3,46 g de produit ayant la référence **200r** (rendement de 33 % calculé à partir de la quantité initiale de **200p.**

4ème étape: Préparation de l'intermédiaire acide 2',5'-diméthyl-4'-(6-acryloyloxyhexyloxy)-4-azophénylcarboxylique (**200s**)

3,00 g (8,10 mmol) de **200r** et 2,40 g (19,8 mmol) de N,N-diméthylaniline sont dissous, en chauffant si nécessaire dans 200 ml de THF (tétrahydrofurane). 0,90 g (9,94 mmol) de chlorure d'acryloyle sont ajoutés à température ambiante. Le mélange est agité deux jours à température ambiante en ajoutant encore 2,40 g (19,8 mmol) de N,N-diméthylaniline et 0,90 g (9,94 mmol) de chlorure d'acryloyle après un jour. Le mélange réactionnel est transvasé dans un entonnoir à séparation, 200 ml d'éther sont ajoutés et la phase organique est lavée avec HCl 2N. La phase organique est séchée et le solvant éliminé à l'évaporateur rotatif. Le résidu est purifié par chromatographie sur gel de silice avec de l'éther comme éluant. La fraction contenant le produit pur est évaporée à sec, donnant 3,16 g de produit ayant la référance **200s** (rendement 92 %), qui peut être recristallisé dans un mélange éther/hexane.

5ème étape: Obtention du produit du titre

2,23 g (5,33 mmol) de **200s** sont dissous dans 200 ml de DME (1,2-diméthoxyéthane) et refroidis à -25 °C. 0,60 g (5,24 mmol) de $CH_3SO_2Cl$ puis 1,10 g (10,87 mmol) de TEA (triéthylamine) sont ajoutés et le mélange est maintenu une heure à -25 °C. 0,36 g (2,46 mmol) de dianhydro-*D*-glucitol et 0,65 g (5,32 mmol) de 4-DMAP sont ajoutés. Le refroidissement est arrêté et le mélange réactionnel est agité encore trois jours à température ambiante avant d'être hydrolysé avec HCl aqueux dilué et transvasé dans un entonnoir à séparation. 200 ml de $CH_2Cl_2$ sont ajoutés puis la phase organique est séparée, séchée sur $MgSO_4$ et le solvant éliminé à l'évaporateur rotatif. Le résidu est purifié par deux chromatographies successives sur gel de silice avec respectivement des mélanges $CH_2Cl_2$/AcOEt (94:6) et $CH_2Cl_2$/AcOEt (96:4) comme éluants. La fraction contenant le produit pur après la deuxième colonne est évaporée à sec donnant 0,78 g d'un solide qui est recristallisé deux fois à chaud dans l'EtOH, donnant 0,24 g de produit du titre (rendement 10 %).

Outre les caractéristiques rapportées dans le tableau 1, on a également effectué le spectre de résonance magnétique nucléaire et le spectre de masse qui confirment la structure et la pureté du composé attendu :
$^1$H-RMN (200 MHz, $CDCl_3$): 1.41-1.61 (*m*, 8H, aliph.); 1.65-1.93 (*m*, 8H, aliph.); 2.54 (*s*, 12H, $CH_3$); 4.03 (*t*, 4H, $OCH_2$); 4.10-4.22 (*m*, 4H, $CO_2CH_2$; 4H, $CO_2CH\underline{H_2}$); 4.74 (*d*, 1H, $CO_2CHC\underline{H}$); 5.12 (*t*, 1 H, $CO_2CHC\underline{H}$); 5.48 (*ddd,* 1H, $CO_2CH$); 5.54 (*m*, 1H, $CO_2CH$); 5.83 (*dd*, 2H, vinyl.); 6.13 (*dd,* 2H, vinyl.); 6.42 (*dd*, 2H, vinyl.); 6.68 (*s*, 4H, arom.); 7.86 (*d*, 4H, arom.); 8.16 (*d*, 2H, arom.); 8.23 (*d*, 2H, arom.).
MS : 959 $(M)^+$.

Exemple 3: 2-[4'-(6-acryloyloxyhexyloxy)-2',5'-méthylazophényl-4-carbonyloyl]-5-[4-(6-acryloyloxyhexyloxy)benzoyl]-1,4;3,6-*D*-glucitol (**209sf**)

Comme on le voit dans la formule développée du tableau 1, ce composé ne présente qu'un seul groupe photo-isomérisable. Pour l'obtenir on modifie les conditions opératoires de la cinquième étape de l'exemple 3, notamment en utilisant un large excès de dianhydro-*D*-glucitol et un temps de réaction plus court, de façon à obtenir d'abord un monoester. Les réactivités différentes des formes *endo* et *exo*, ainsi que leurs comportements différents lors des chromatographies sur gel de silice permettent d'obtenir sélectivement la forme exo qui sera utilisée dans le présent exemple ou la forme endo qui sera utilisée, dans les deux exemples suivants.

1ère étape: Obtention des intermédiaires **209s-monoester**-*exo* et **209s-monoester**-*endo*

4,15 g (9,78 mmol) de **200s** sont dissous dans 200 ml de DME et refroidis à -30 °C. 1,21 g (10,56 mmol) de $CH_3SO_2Cl$ puis 2,12 g (20,95 mmol) de TEA sont ajoutés et le mélange est maintenu 1h30 à -30 °C. Une solution de 9,5 g (65,00 mmol) de dianhydro-*D*-glucitol dans 50 ml de DME puis 1,30 g (10,64 mmol) de 4-DMAP sont ajoutés. Le refroidissement est arrêté et le mélange réactionnel est agité une nuit à température ambiante avant d'être hydrolysé avec HCl aqueux dilué et transvasé dans un entonnoir à séparation. La phase aqueuse est extraite avec $CH_2Cl_2$ jusqu'à sa décoloration. Les phases organiques sont regroupées, séchées sur $MgSO_4$ et le solvant éliminé à l'évaporateur rotatif donnant 6,0 g d'un solide qui est purifié par chromatographie sur gel de silice.

Une première fraction avec $CH_2Cl_2$/AcOEt (95:5) comme éluant est collectée contenant entre autres le produit de la diestérification **209s**. Une deuxième fraction avec $CH_2Cl_2$/AcOEt (85:15) comme éluant est collectée, évaporée à sec et le solide ainsi obtenu est recristallisé à chaud dans l'hexane, donnant 1,30 g de **209s**-monoester-*exo* (rendement 24 %).

Le spectre de résonance magnétique nucléaire de ce composé est le suivant:
$^1$H-RMN (200 MHz, $CDCl_3$): 1.41-1.62 (*m*, 4H, aliph.); 1.68-1.93 (*m*, 4H; aliph.); 2.53 (*s*, 6H, $CH_3$); 3.64 (*dd*, 1 H, CH(OH)C$\underline{H}_2$); 3.96 (*dd,* 1H, CH(OH)C$\underline{H}_2$); 4.02 (*t*, 2H, OC$\underline{H}_2$$CH_2$); 4.10-4.22 (*m*, 2H, $CO_2$$CH_2$; 2H, $CO_2$CHC$\underline{H}_2$); 4.37 (*ddd,* 1H, C$\underline{H}$OH); 4.67 (*d*, 1H, $CO_2$CHC$\underline{H}$); 4.75 (*t*, 1 H, CH(OH)C$\underline{H}$); 5.51 (*d*, 1H, $CO_2$CH); 5.82 (*dd*, 1H, vinyl.); 6.12 (*dd*, 1H, vinyl.); 6.41 (*dd,* 1 H, vinyl.); 6.67 (*s*, 2H, arom.); 7.86 (*d*, 2H, arom.); 8.15 (*d*, 2H, arom.).

Une troisième fraction avec $CH_2Cl_2$/AcOEt (75:25) comme éluant est collectée, évaporée à sec et le solide ainsi obtenu est recristallisé à chaud dans l'hexane pour donner 2,59 g de **209s-monoester**-*endo* (rendement 48 %).

Le spectre de résonance magnétique nucléaire de ce composé est le suivant:
$^1$H-RMN (200 MHz, $CDCl_3$): 1.42-1.61 (*m*, 4H, aliph.); 1.66-1.86 (*m*, 4H, aliph.); 2.54 (*s*, 6H, $CH_3$); 3.93-4.05 (*m*, 2H, OC$\underline{H}_2$$CH_2$; 2H, $CO_2$CHC$\underline{H}_2$; 2H, CH(OH)C$\underline{H}_2$); 4.19 (*t*, 2H, $CO_2$$CH_2$); 4.39 (*m*, 1 H, C$\underline{H}$OH); 4.47 (*d*, 1 H, CH(OH)C$\underline{H}$); 5.02 (*t*, 1 H, $CO_2$CHC$\underline{H}$); 5.43 (*ddd,* 1 H, $CO_2$CH); 5.82 (*dd*, 1 H, vinyl.); 6.13 (*dd,* 1 H, vinyl.); 6.41 (*dd*, 1 H, vinyl.); 6.67 (*s*, 2H, arom.); 7.87 (*d*, 2H, arom.); 8.19 (*d*, 2H, arom.).

2ème étape: Obtention du composé du titre

3,67 g (12,55 mmol) d'acide 4-(6-acryloyloxyhexyloxy)benzoïque sont dissous dans 150 ml de DME et refroidis à -25 °C. 1,42 g (12,40 mmol) de $CH_3SO_2Cl$ puis 2,54 g (25,10 mmol) de TEA sont ajoutés et le mélange est maintenu une heure à -25 °C. 3,34 g (6,04 mmol) de **209s-monoester-***exo* préparé à l'étape précédente dissous dans 30 ml de DME puis 0,76 g (6,22 mmol) de 4-DMAP sont ajoutés. Le refroidissement est arrêté et le mélange réactionnel est agité encore une nuit à température ambiante avant d'être chauffé à reflux durant 6 heures. Après refroidissement, le mélange réactionnel est hydrolysé avec HCl aqueux dilué et transvasé dans un entonnoir à séparation. La phase aqueuse est extraite avec $CH_2Cl_2$ jusqu'à ce que la phase aqueuse soit incolore. Les phases organiques sont groupées, séchées sur $MgSO_4$ et le solvant éliminé à l'évaporateur rotatif, donnant un résidu qui est purifié par chromatographie sur silice avec un éluant $CH_2Cl_2$/AcOEt (96:4). La fraction contenant le produit pur est évaporée à sec et le solide recristallisé a chaud dans l'EtOH, donnant 3,65 g de produit du titre (rendement 73%).

Outre les caractéristiques rapportées dans le tableau 1, on a effectué le spectre de résonance magnétique nucléaire et le spectre de masse qui confirment la pureté et la structure du produit attendu :
$^1$H-RMN (200 MHz, $CDCl_3$): 1.42-1.60 (*m*, 8H, aliph.); 1.67-1.95 (*m*, 8H, aliph.); 2.54 (*s*, 6H, $CH_3$); 4.00-4.22 (*m*, 4H, $CO_2$$CH_2$; 4H, OC$\underline{H}_2$$CH_2$; 4H, $CO_2$CHC$\underline{H}_2$); 4.72 (*d*, 1H, $CO_2$CHC$\underline{H}$); 5.09 (*t*, 1H, $CO_2$CHC$\underline{H}$); 5.43 (*ddd*, 1H, $CO_2$CH); 5.52 (*m*, 1H, $CO_2$CH); 5.83 (*dd*, 2H, vinyl.); 6.13 (*m*, 2H, vinyl.); 6.42 (*m*, 2H, vinyl.); 6.68 (*s*, 2H, arom.); 6.93 (*d*, 2H, arom.); 7.86 (*d*, 2H, arom.); 8.04 (*d*, 2H, arom.); 8.15 (*d*, 2H, arom.).
MS : 827 $(M+H)^+$.

Exemple 4: 2-[4-(6-acryloyloxyhexyloxy)benzoyl]-5-[4'-(6-acryloyloxyhexyloxy)-2',5'-méthylazophenyl-4-carbonyloyl]-1,4;3,6-*D*-glucitol (**209fs**)

4,40 g (15,05 mmol) d'acide 4-(6-acryloyloxyhexyloxy)benzoïque sont dissous dans 200 ml de DME et refroidis à -25 °C. 1,70 g (14,84 mmol) de $CH_3SO_2Cl$ puis 3,04 g (30,04 mmol) de TEA sont ajoutés et le mélange est maintenu une heure à -25 °C. 4,01 g (7,26 mmol) de **209s-monoester**-*endo* dissous dans 30 ml de DME puis 0,88 g (7,20 mmol) de 4-DMAP sont ajoutés. Le refroidissement est arrêté et le mélange réactionnel est agité encore une nuit à température ambiante avant d'être chauffé à reflux durant 6 heures. Après refroidissement, le mélange est hydrolysé avec HCl aqueux dilué et transvasé dans un entonnoir à séparation. La phase aqueuse est extraite avec $CH_2Cl_2$ jusqu'à ce que la phase aqueuse soit incolore. Les phases organiques sont groupées, séchées sur $MgSO_4$ et le solvant éliminé à l'évaporateur rotatif, donnant un résidu qui est purifié par chromatographie sur silice avec un éluant $CH_2Cl_2$/AcOEt (96:4). La fraction contenant le produit pur est évaporée à sec et le solide recristallisé a chaud dans l'EtOH, donnant 4,21 g de produit du titre (rendement 70%).

Outre les caractéristiques rapportées dans le tableau 1, on a effectué le spectre de résonance magnétique nucléaire qui confirme la pureté et la structure du produit attendu :
$^1$H-RMN (200 MHz, $CDCl_3$): 1.42-1.62 (*m*, 8H, aliph.); 1.67-1.95 (*m*, 8H, aliph.); 2.54 (*s*, 6H, $CH_3$); 3.98-4.22 (*m*, 4H, $CO_2$$CH_2$; 4H, OC$\underline{H}_2$$CH_2$; 4H, $CO_2$CHC$\underline{H}_2$); 4.69 (*d*, 1H, $CO_2$CHC$\underline{H}$); 5.08 (*t*, 1H, $CO_2$CHC$\underline{H}$); 5.42-5.50 (*m*, 2H, $CO_2$CH); 5.82 (*dd*, 2H, vinyl.); 6.12 (*m*, 2H, vinyl.); 6.41 (*m*, 2H, vinyl.); 6.68 (*s*, 2H, arom.); 6.90 (*d*, 2H, arom.); 7.88 (*d*, 2H, arom.); 7.96 (*d*, 2H, arom.); 8.21 (*d*, 2H, arom.).

Exemple 5: 2-[4'-(6-acryloyloxyhexyloxy)-2',5'-méthylazophényl-4-carbonyloyl]-5-acrylyloyl-1,4;3,6-*D*-glucitol (**209sa**)

Comme les composés des exemples 3 ou 4, ce composé ne comporte qu'un groupe photoisomérisable, mais en diffère en ce que la chaîne non photoisomérisable (mais polymérisable) est plus courte. Il est obtenu par l'action

de l'acide acrylique sur le **monoester-*endo*** (**209s-monoester**-*endo*) du dianhydro-*D*-glucitol, comme expliqué ci-après.

1,95 g (3,53 mmol) de **209s-monoester-**endo sont dissous dans 40 ml de THF. 2,40 g (19,8 mmol) de N,N-diméthylaniline puis 1,00 g (11,0 mmol) de chlorure d'acryloyle sont ajoutés à température ambiante. Le mélange est agité à température ambiante durant 5 jours. Le mélange réactionnel est concentré à 5-10 ml en prenant garde de ne pas trop chauffer, puis une chromatographie rapide est effectuée avec $CH_2Cl_2$/AcOEt (95:5) comme éluant dans le but d'éliminer l'excès de chlorure d'acryloyle et diminuer ainsi les risques de polymérisation. Une fraction est collectée qui est lavée avec NaOH 1 N puis HCl 1 N. Après séchage sur $MgSO_4$, le solvant est éliminé à l'évaporateur rotatif, donnant un résidu qui est purifié immédiatement par chromatographie sur silice. Une première fraction avec $CH_2Cl_2$ comme éluant est collectée contenant des impuretés. La fraction avec le produit désiré est éluée avec $CH_2Cl_2$/AcOEt (95:5) comme éluant. Après élimination du solvant, une huile est obtenue qui est cristallisée après dissolution à chaud dans l'EtOH, donnant 1,0 g de produit du titre (rendement 47%).

Outre les caractéristiques rapportées dans le tableau 1, on a effectué un spectre de résonance magnétique nucléaire qui confirme la structure et la pureté du produit attendu :
$^1$H-RMN (200 MHz, $CDCl_3$): 1.42-1.92 (*m*, 8H, aliph.); 2.55 (s, 6H, $CH_3$); 4.00-4.22 (*m*, 2H, $CO_2CH_2$; 2H, $OC\underline{H_2}CH_2$; 4H, $CO_2CHC\underline{H_2}$); 4.61 (*d*, 1 H, $CO_2CHC\underline{H}$); 5.04 (*t*, 1H, $CO_2CHC\underline{H}$); 5.34 (*m*, 1H, $CO_2CH$); 5.45 (*ddd*, 1H, $CO_2CH$); 5.83 (*dd*, 2H, vinyl.); 6.13 (*m*, 2H, vinyl.); 6.42 (*m*, 2H, vinyl.); 6.68 (*s*, 2H, arom.); 7.88 (*d*, 2H, arom.); 8.20 (*d*, 2H, arom.).

Exemple 6: 2-[4'-(6-acryloyloxyhexyloxy)-2',5'-méthylazophényl-4-carbonyloyl]-5-[4-(6-acryloyloxyhexyloxy)benzoyl]-1,4;3,6-*D*-mannitol (**210 fs**)

Ce composé, qui est un diastéréoisomère du composé décrit à l'exemple 3, est préparé à partir du 1,4; 3,6-dianhydro-*D*-mannitol avec quelques modifications du processus expérimental. Le schéma de synthèse n'est pas repris dans la figure 1.

1$^{ère}$ étape: Préparation de l'intermédiaire 1,4;3,6-dianhydro-*D*-mannitol monosubstitué (**110f-monoester**)

2,00 g (6,84 mmol) d'acide 4-(6-acryloyloxyhexyloxy)benzoïque sont dissous dans 150 ml de DME et refroidis à -25 °C. 0,80 g (6,84 mmol) de $CH_3SO_2Cl$ puis 1,40 g (13,83 mmol) de TEA sont ajoutés et le mélange est maintenu une heure à -25 °C. Une solution de 3,00 g (20,53 mmol) de 1,4:3,6-dianhydro-D-mannitol dans 50 ml de DME puis 2,50 g (20,46 mmol) de 4-DMAP sont ajoutés. Le refroidissement est arrêté et le mélange réactionnel est agité encore un jour à température ambiante avant d'être hydrolysé avec HCl aqueux dilué et transvasé dans un entonnoir à séparation. 300 ml de $CH_2Cl_2$ sont ajoutés et la phase organique est séparée, séchée sur $MgSO_4$ puis le solvant éliminé à l'évaporateur rotatif, entraînant la polymérisation d'une partie du produit. Le reste est dissous rapidement dans $CH_2Cl_2$ puis purifié par chromatographie sur silice avec un éluant $CH_2Cl_2$/AcOEt (75:25). La fraction contenant le produit pur est évaporée à sec et le solide recristallisé à chaud dans l'EtOH entraînant à nouveau la polymérisation d'une partie du produit. Le polymère est éliminé par filtration et la solution est laissée à cristalliser à température ambiante puis au réfrigérateur, donnant finalement 0,84 g de produit ayant la référence **110f-monoester** (rendement 29 %).

2$^{ème}$ étape: Obtention du composé du titre

420 mg (0,99 mmol) du composé préparé à la quatrième étape de l'exemple 1 (**200s**) sont dissous dans 100 ml de DME et refroidis à -25°C. 113 mg (0,99 mmol) de $CH_3SO_2Cl$ puis 200 mg (1,98 mmol) de TEA sont ajoutés et le mélange est maintenu une heure à -25 °C. 800 mg (1,90 mmol) de l'intermédiaire **110f-monoester** préparé dans la première étape et 121 mg (0,99 mmol) de 4-DMAP sont ajoutés. Le refroidissement est arrêté et le mélange réactionnel est agité encore un jour à température ambiante avant d'être hydrolysé avec HCl aqueux dilué et transvasé dans un entonnoir à séparation. 400 ml d'éther sont ajoutés puis la phase organique est séparée, séchée sur $MgSO_4$ et le solvant éliminé à l'évaporateur rotatif. Le résidu obtenu est purifié une première fois par chromatographie sur gel de silice avec un éluant $CH_2Cl_2$/AcOEt (90:10) puis une deuxième fois avec un éluant $CH_2Cl_2$/AcOEt (95:5). La fraction contenant le produit pur est évaporée à sec, donnant 109 mg de composé du titre (rendement 13 %).

Outre les caractéristiques rapportées dans le tableau 1, on a effectué le spectre de résonance magnétique nucléaire et le spectre de masse qui confirment la pureté et la structure du produit attendu:
$^1$H-RMN (200 MHz, $CDCl_3$): 1.41-1.61 (*m*, 8H, aliph.); 1.65-1.93 (*m*, 8H, aliph.); 2.54 (*s*, 6H, $CH_3$); 4.02 (*t*, 4H, $OC\underline{H_2}CH_2$); 4.10-4.27 (*m*, 4H, $CO_2CH_2$; 4H, $CO_2CHC\underline{H_2}$); 4.87-4.95 (*m*, 2H, $CO_2CHC\underline{H}$); 5.29-5.43 (*m*, 2H, $CO_2CH$); 5.83 (*dd*, 2H, vinyl.); 6.13 (*dd*, 2H, vinyl.); 6.42 (*dd*, 2H, vinyl.); 6.68 (*s*, 2H, arom.); 6.92 (*d*, 2H, arom.); 7.88 (*d*, 2H, arom.); 8.05 (*d*, 2H, arom.); 8.23 (*d*, 2H, arom.).
MS : 827 $(M+H)^+$.

Les expériences réalisées, rapportés dans les exemples 7 à 8 ci-après, montrent que les dopants chiraux

selon l'invention permettent de modifier les propriétés optiques d'un milieu, et notamment d'induire dans un milieu cristal liquide nématique une hélicité variable en fonction de la nature et de la durée d'une irradiation dont la longueur d'onde peut aller du visible (VIS) à l'ultraviolet (UV). Dans une application préférée, les propriétés des dopants chiraux selon l'invention sont mises en oeuvre pour réaliser un affichage trichrome.

Exemple 7: Rotation spécifique, solubilité et HTP

Pour l'application sus-indiquée les composés doivent posséder une solubilité et un HTP élevé dans un nématique. Ces valeurs, pour un certain nombre de composés selon l'invention sont rapportées dans le tableau 2 ci-après. Dans ce tableau on a également rapporté, avant et après isomérisation, la valeur de la rotation spécifique $[\alpha]^{20}_{\lambda}$ pour les concentrations indiquées dans le chlorure de méthylène, et pour les longueurs d'onde mentionnées.

Tableau 2

| Composé | Concentration g/100ml $CH_2Cl_2$) | | | Solubilité s (% masse) | HTP ($\mu m^{-1}$) |
|---|---|---|---|---|---|
| | | $[\alpha]^{20}_{\lambda}$ avant iso. | après iso. | | |
| **201c** | 0,06 | - 64°$_{546}$ | - 135°$_{546}$ | 1,5% < s < 3,0% | 20 |
| **201e** | 0,1 | - 60°$_{546}$ | - 114°$_{546}$ | 1,5% < s < 3,0% | 19 |
| **206c** | 0,09 | - 222°$_{546}$ | - 81°$_{546}$ | 0,6% < s < 0,8% | 13 |
| **206e** | 0,09 | - 177°$_{546}$ | - 19°$_{546}$ | 3,0% < s < 4,0% | 11 |
| **209s** | 0,05 | - 248°$_{578}$ | - 129°$_{578}$ | 3,0% < s < 3,9% | 35 |
| **209sf** | 0,06 | - 85°$_{578}$ | - 47°$_{578}$ | 4,0% < s | 35 |
| **210fs** | 0,06 | + 221°$_{578}$ | + 198°$_{578}$ | 1,9% < s < 6,0% | 17 |

A l'exception du composé 210fs, on observera que les autres composés montrent une modification remarquable de leur rotation optique, le plus souvent supérieure à 50% ce qui montre leur aptitude d'isomérisation par irradiation. Ce tableau confirme par ailleurs ce qui était déjà connu, à savoir l'absence de corrélation évidente entre la variation de la rotation spécifique et la valeur HTP des composés. Ce tableau montre que, pour la majorité des composés étudiés la solubilité est de l'ordre de 3 % en poids ou supérieure et que le HTP varie entre 11 $\mu m^{-1}$ et 35 $\mu m^{-1}$.

Exemple 8: Photomodulation de la couleur de cellules d'affichage

A titre d'exemple, pour les expériences rapportées ci-après on a retenu le dopant chiral **209s** qui présente un HTP de 35 $\mu m^{-1}$ assez élevé et une bonne solubilité permettant de l'incorporer à une concentration de 3,3 % dans un matériau cholestérique de base, comprenant notamment un mélange nématique formé de biphényles (par exemple disponible chez Merck sous la référence E48). La composition ainsi préparée est introduite dans une cellule d'essai comprenant un substrat avant et un substrat arrière, ces substrats étant formés par des plaques de verre, de 2,2 x 2,8 mm et de 0,3 mm d'épaisseur maintenues par un cadre de scellement à un écartement de 6 μm. La cellule comporte une couche d'alignement planaire non frottée, ainsi qu'un pixel carré de 1 cm de côté comportant sur chacun des substrats un revêtement en oxyde d'indium et d'étain (ITO) formant les électrodes qui permettront de commuter la cellule d'un état dans un autre par application d' un champ électrique. Une fois remplie, la cellule est exposée à une irradiation UV et on suit l'évolution du spectre UV-VIS en fonction du temps d'exposition.

Dans l'expérience correspondant au graphique de la figure 3, donnant le pourcentage de transmission en fonction de la longueur d'onde, seul le dopant chiral 209s a été ajouté au matériau cholestérique de base et on a représenté l'état initial (courbe a) et le spectre respectivement après 1 minute (courbe b), 3 minutes (courbe c) et 10 minutes d'exposition (courbe d). Un temps d'exposition plus long ne modifie plus le spectre, la courbe d représentant le spectre de la cellule photomodulée au maximum. On observe alors un déplacement atteignant 160 nm à partir du spectre initial, c'est à dire un déplacement allant du bleu violet (courbe b) au rouge orangé (courbe d), en passant par

le vert (courbe c).

Dans l'expérience correspondant au graphique de la figure 4, on a ajouté au matériau cholestérique de base 3 % en poids d'un photoinitiateur de type morpholinocétone commercialement disponible chez Ciba-Geigy sous la référence Irgacure® 369. Ce photoinitiateur vise à améliorer la polymérisation des enchaînements terminaux. En vu d'améliorer la polymérisation, et donc la fixation d'une couleur déterminée, on peut également remplacer le photoinitiateur par un comonomère de type diacrylate, tel que le composé RM-82 disponible chez Merck. Il est également possible d'utiliser simultanément, dans des rapports appropriés, à la fois un photoinitiateur et un comonomère. En se référant maintenant à la figure 4 qui concerne une cellule dont le matériau cholestérique de base ne contient qu'un dopant selon l'invention et un photoinitiateur. On a également reporté quatre courbes représentant respectivement l'état initial (courbe a), le spectre après 2 minutes (courbe b), 5 minutes (courbe c) et 18 minutes (courbe d). On peut alors observer une variation de 190 nm entre le spectre initial et le spectre de la cellule photomodulée au maximum.

En remplaçant dans l'expérience ci-dessus le dopant chiral 209s par le dopant chiral **209sf** à la concentration de 4,1 % en poids, on observerait dans les mêmes conditions au bout de 5 minutes une variation de 220 nm.

Exemple 9: Premier mode de réalisation d'une cellule trichrome

Le composé **209sf** (exemple 4) ayant montré une très bonne aptitude à modifier le pas d'hélice du matériau cholestérique avec une fenêtre de 220 nm a été retenu pour la préparation de cellules d'affichage multicouleurs. A partir de cellules identiques à celles utilisées dans l'exemple 8 ne comportant aucun cloisonnement et contenant un seul et même matériau monochrome, on a réalisé une cellule avec trois zones de couleur différente (bleu, vert et rouge) en effectuant un masquage sélectif ou progressif des trois zones permettant de moduler leur taux d'irradiation respectif.

De telles cellules trichromes peuvent soit permettre l'affichage d'informations, soit être utilisées comme filtre coloré dans un ensemble d'affichage.

Dans un processus dit "dissociatif", chaque famille de pixels est photomodulée individuellement, les deux autres familles de pixels étant recouvertes d'un masque faisant barrière au rayonnement, le processus étant reproduit pour chacune des trois familles en faisant varier le temps d'irradiation de chaque famille.

Bien entendu, une cellule d'affichage comportant un très grand nombre de pixels de très petites dimensions, les opérations de masquage sont effectuées au moyen de masques formés ou obtenus par des procédés connus telle que la technique de photolithographie.

Ce processus est schématiquement représenté à la figure 5. On voit que, partant d'une composition chimique d'un matériau cholestérique CM identique pour tous les pixels, on crée dans une première étape 1 une famille de pixels bleus (B), puis dans une deuxième étape 2 une famille de pixels verts (G), et dans une troisième étape 3 une famille de pixels rouges (R) pour former un affichage trichrome CD.

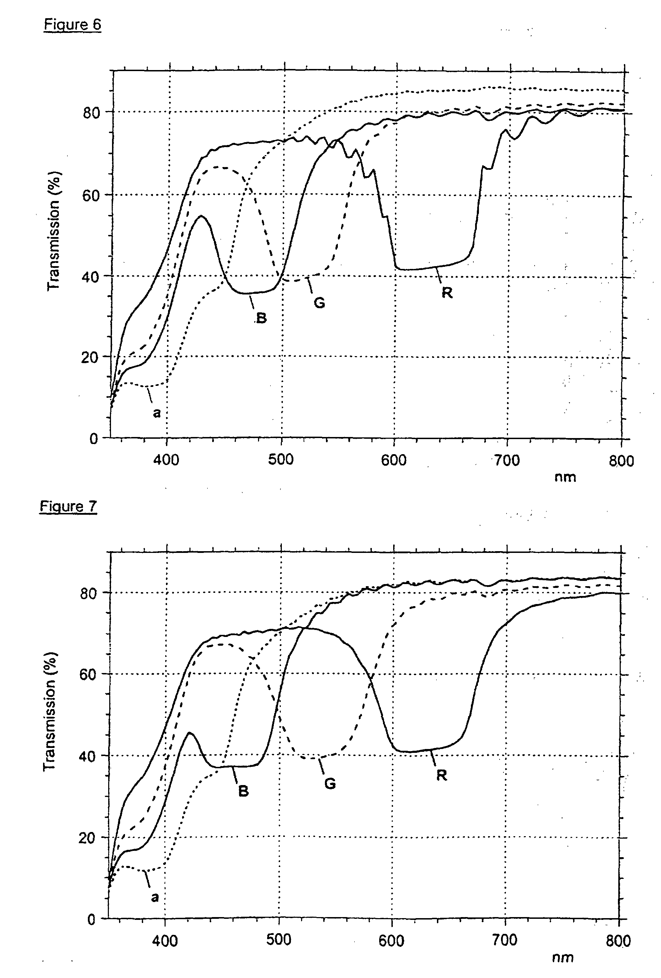

Le graphique de la figure 6 donne le pourcentage de transmission en fonction de la longueur d'onde, à l'état initial (courbe a), pour les pixels de couleur bleue (courbe B), pour les pixels de couleur verte (courbe G) et pour les pixels de couleur rouge (courbe R).

Exemple 10 : Deuxième mode de réalisation d'une cellule trichrome

Dans ce deuxième mode de réalisation la pixelisation est réalisée selon un processus dit "additif". La cellule toute entière, c'est-à-dire tous les pixels devant définir les trois couleurs, est soumise à une première période d'exposition, puis une famille de pixels est masquée et on poursuit l'exposition pendant une deuxième période et enfin une deuxième famille de pixels différente de la précédente est à son tour masquée et on termine l'exposition pendant une troisième période.

Avec une cellule ayant les mêmes caractéristiques que celles de l'exemple 9, et pour des périodes d'exposition identiques pour chaque famille de pixels on a obtenu le graphique donnant le pourcentage de transmission en fonction de la longueur d'onde représenté à la figure 7.

En comparant les graphiques des figures 6 et 7, on observera qu'il n'existe pas de différences significatives entre le processus dit "dissociatif" et le processus dit "additif".

Exemple 11 : Troisième mode de réalisation d'une cellule trichrome

Dans les modes de réalisation des exemples 9 et 10 ci-dessus, on a indiqué qu'il n'existait aucun cloisonnement entre le substrat inférieur et le substrat supérieur pour délimiter les pixels individuels. Ces modes de réalisation sont satisfaisants lorsqu'on peut obtenir une forte polymérisation du milieu. Dans le cas contraire, et selon le troisième mode de réalisation, il est possible de prévoir une structuration du substrat inférieur en "nid d'abeille" ou en canaux, qui permettra, après remplissage avec une unique composition d'un matériau cholestérique selon l'invention, d'isoler physiquement les pixels les uns des autres. Bien entendu, la photomodulation de chaque famille de pixels peut être

obtenu selon un processus "dissociatif" ou selon un processus "additif", comme indiqué précédemment.

Exemple 12 : Quatrième mode de réalisation d'une cellule trichrome

Dans les modes de réalisation des exemples 9 à 11, l'utilisation de pixels permet d'avoir un affichage en couleur, c'est-à-dire la perception par l'observateur d'une image colorée par fusionnement des pixels d'un même point image. Le quatrième mode de réalisation constitue en quelque sorte une simplification en ce que la surface d'affichage est divisée en plusieurs zones photomodulables ayant chacune une sous-famille de pixels permettant d'afficher une information dans une unique couleur déterminée, et de différencier ainsi très simplement des informations de catégories différentes. Ce mode de réalisation n'exclut nullement de prévoir une zone réalisée selon l'un des trois autres mode de réalisation.

## Revendications

1. Dopants chiraux permettant d'induire une modification du pas d'hélice d'une composition cristal liquide cholestérique ayant une structure chimique comprenant une unité chirale bifonctionnelle, dont au moins une des fonctions permet d'établir une liaison chimique avec un groupe isomérisable, ledit groupe possédant éventuellement un enchaînement terminal polymérisable ou copolymérisable, **caractérisés en ce qu'**ils répondent à la formule I suivante

$$A_1\text{-}Y_1\text{-}X\text{-}Y_2\text{-}A_2 \qquad (I)$$

dans laquelle

- X représente un radical chiral dérivant d'un composé bifontionnalisé,
- $Y_1$ et $Y_2$ sont identiques ou différents et représentent chacun une unité de liaison fonctionnelle choisie parmi -O-, -S-, -COO-, -OCO-, -CON(R)- et
- N(R)CO- dans laquelle R représente un reste alkyle.
- $A_1$ représente un groupe mésogénique photoisomérisable correspondant à la formule (II).

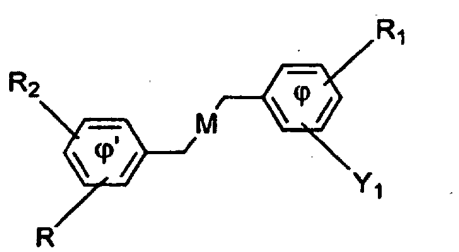
(II)

dans laquelle deux restes phenyle φ et φ' sont liés par un radical bivalent photoisomérisable -M- choisi parmi -N=N-, -N=CH-, -CH=N- et -CH=CH-; lesdits restes phényle ayant respectivement un ou plusieurs substituants $R_1$, $R_2$ identiques ou différents choisis parmi l'hydrogène, les radicaux alkyle ou alkoxy de $C_1$ à $C_5$, les halogènes, et les radicaux cyano, nitro, ou trifluorométhyle; et R' représente l'hydrogène ou un groupe correspondant à la formule (III)

$$-Y_3\text{-}C_nH_{2n}\text{-}Z \qquad (III)$$

dans laquelle $Y_3$ a les mêmes significations que $Y_1$ et $Y_2$ ou représente le phénylène, n est un nombre entier compris entre 0 et 12 et Z représente l'hydrogène ou un reste polymérisable choisi parmi les restes acryloyl et acryloyloxy; et

- $A_2$ a la même signification que les groupes de formule (II) ou de formule (III).

**2.** Dopants chiraux selon la revendication 1, **caractérisés en ce que** le radical bivalent -M -est le radical azo -N=N- pour former avec les restes φ et φ' un radical azophényl et **en ce que** les subsistants R et $Y_1$ ou $Y_2$ occupent les mêmes positions ortho, meta ou para respectivement sur les restes φ et φ'

**3.** Dopants chiraux selon la revendication 1, **caractérisés en ce que** $A_1$ et $A_2$ correspondent chacun à un groupe mésogénique photoisomérisable de formule II.

**4.** Dopants chiraux selon la revendication 1, **caractérisés en ce que** $A_2$ correspond au groupe de formule III.

**5.** Dopants chiraux selon l'une quelconque des revendications 1 à 4, **caractérisés en ce que** R représente un groupe de formule III et **en ce que** Z représente toujours un radical polymérisable choisi parmi les radicaux acryloyl et acryloyloxy.

**6.** Dopants chiraux selon la revendication 1, **caractérisés en ce que** le radical chiral X est dérivé d'un diacide ou d'un diol chiral.

**7.** Dopants chiraux selon l'une quelconque des revendications 1 à 6, **caractérisés en ce que** le radical chiral X correspond aux radicaux bifonctionnels dérivés du dianhydro-glucitol ou du dianhydro-mannitol.

**8.** Dopant chiral selon la revendication 7, **caractérisé en ce qu'**il correspond au composé suivant:

-2,5-Bis[4'-(6-acryloyloxyhexyloxy)-2',5'-méthylazophényl-4-carbonyloyl]-1,4;3,6-dianhydro-*D*-glucitol.

**9.** Dopant chiral selon la revendication 7, **caractérisé en ce qu'**il correspond à la formule suivante:

-2-[4'-(6-acryloyloxyhexyloxy)-2',5'-méthylazophényl-4-carbonyloyl]-5-[4-(6-acryloyloxyhexyloxy)benzoyl]-1,4;3,6-*D*-glucitol.

**10.** Procédé de modulation des dopants chiraux selon la revendication 1, **caractérisé en ce que** la modification de l'induction du pas d'hélice desdits dopants chiraux est obtenue par irradiation ultraviolette (UV), visible (VIS) ou mixte (UV + VIS) agissant sur le groupe isomérisable, le temps d'irradiation étant contrôlé pour définir une couleur déterminée lorsque lesdits dopants sont incorporés dans une composition cristal liquide cholestérique.

**11.** Cellule d'affichage comprenant deux substrats transparents, supportant des électrodes reliées à un circuit électronique, et un cadre de scellement délimitant ensemble un espace lamellaire dans lequel est introduite une composition cristal liquide cholestérique incorporant au moins un dopant chiral selon la revendication 1, **caractérisé en ce que** la cellule est soumise à une irradiation ultraviolette (UV) ou visible (VIS) modulée en fonction de différentes zones, ou de différentes familles de pixels en utilisant des masques et en faisant varier le temps d'irradiation.

**12.** Cellule d'affichage selon la revendication 11, **caractérisée en ce que** chaque famille de pixels correspond à un masque déterminé permettant d'ajuster le temps d'irradiation de chaque famille.

**13.** Cellule d'affichage selon la revendication 11, **caractérisée en ce que** des masques sont conçus pour découvrir successivement chaque famille de pixels et permettre leur irradiation progressive.

**14.** Cellule d'affichage selon la revendication 11, **caractérisée en ce qu'**elle ne comporte aucun cloisonnement, et **en ce que** les dopants chiraux selon la revendication 1 possèdent un enchaînement terminal polymérisable ou copolymérisable.

**15.** Cellule d'affichage selon la revendication 11, **caractérisée en ce qu'**elle comporte des cloisonnements pour chaque zone ou pour chaque pixel, et **en ce que** les dopants chiraux selon la revendication 1 peuvent ne pas posséder un enchaînement terminal polmérisable ou copolymérisable.

## Claims

**1.** Chiral doping agents allowing modification in the spiral pitch of a cholesteric liquid crystal composition to be induced having a chemical structure including a biactivated chiral unit, at least one of whose functions allows a chemical

link to be established with an isomerisable group, said group possibly having a polymerisable or co-polymerisable end chain, **characterised in that** they answer the following formula 1:

$$A_1\text{-}Y_1\text{-}X\text{-}Y_2\text{-}A_2 \quad \text{(I)}$$

wherein

- X represents a chiral radical derived from a biactivated compound,
- $Y_1$ and $Y_2$ are identical or different and each represent a functional linking unit selected from among -O-, -S-, -COO-, -OCO-, -CON(R)- and -N(R)CO-

where R represents an alkyl residue,

- $A_1$ represents a photoisomerisable mesogenic group corresponding to the formula (II):

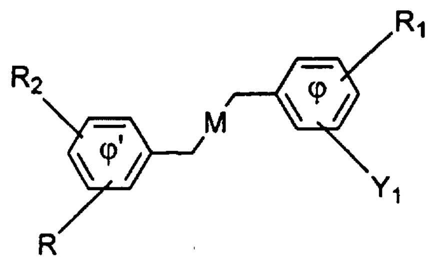

(II)

wherein two phenyl residues φ and φ' are linked by a photoisomerisable -M- bivalent radical selected from among -N=N-, -N=CH-, -CH=N- and -CH=CH- ; said phenyl residues having respectively one or more substituents $R_1$, $R_2$ which are different or identical selected from among hydrogen, the alkyl or alkoxy radicals from $C_1$ to $C_5$, the halogens, and the cyano, nitro or trifluoromethyl radicals; and R' represents hydrogen or a group corresponding to the formula (III):

$$\text{-}Y_3\text{-}C_nH_{2n}\text{-}Z \quad \text{(III)}$$

wherein $Y_3$ has the same significance as $Y_1$ and $Y_2$ or represents phenylene, n is an integer number comprised between 0 and 12 and Z represents hydrogen or a polymerisable moiety selected from among the acryloyl and acryloyloxy moieties; and

- A2 has the same significance as the groups of formula (II) or formula (III).

**2.** Chiral doping agents according to claim 1, **characterised in that** the -M- bivalent radical is the radical azo-N=N- to form with the moieties φ and φ' an azophenyl radical and **in that** the substituents R and $Y_1$ or $Y_2$ occupy the same ortho, meta or para positions respectively on moieties φ and φ'.

**3.** Chiral doping agents according to claim 1, **characterised in that** $A_1$ and $A_2$ each correspond to a photoisomerisable mesogenic group of formula II.

**4.** Chiral doping agents according to claim 1, **characterised in that** $A_2$ corresponds to the group of formula III.

**5.** Chiral doping agents according to any one of claims 1 to 4, **characterised in that** R represents a group of formula III and **in that** Z still represents a polymerisable radical selected from among the acryloyl and acryloyloxy radicals.

**6.** Chiral doping agents according to claim 1, **characterised in that** the chiral radical agent X is derived from a diacide or a chiral diol.

**7.** Chiral doping agents according to any one of claims 1 to 6, **characterised in that** the chiral radical X corresponds to the biactivated radicals derived from dianhydro-glucitol or dianhydro-mannitol.

**8.** Chiral doping agents according to claim 7, **characterised in that** it corresponds to the following compound:

2,5-Bis [4'-(6-acryloyloxyhexyloxy)-2',5'-méthylazophényl-4-carbonyloyl]-1,4;3,6-dianhydro-*D*-glucitol.

**9.** Chiral doping agents according to claim 7, **characterised in that** it corresponds to the following formula:

2- [4'-(6-acryloyloxyhexyloxy)-2',5'-méthylazophényl-4-carbonyloyl]-5-[4-(6-acryloyloxyhexyloxy)benzoyl]-1,4;3,6-*D*-glucitol.

**10.** Method for modulating chiral doping agents according to claim 1, **characterised in that** the modification to the spiral pitch induction of said chiral doping agents is obtained by ultraviolet (UV), visible (VIS) or mixed (UV + VIS) radiation acting on the isomerisable group, the radiation time being controlled to define determined colour when said doping agents are incorporated in a cholesteric liquid crystal composition.

**11.** Display cell including two transparent substrates, supporting electrodes connected to an electronic circuit, and a sealing frame delimiting together a lamellar space into which a cholesteric liquid crystal composition is introduced incorporating at least a chiral doping agent according to claim 1, **characterised in that** the cell is subjected to ultraviolet (UV) or visible (VIS) radiation modulated as a function of different zones, or different pixel families by using masks and varying the radiation time.

**12.** Display cell according to claim 11, **characterised in that** each family of pixels corresponds to a determined mask allowing the radiation time of each family to be adjusted.

**13.** Display cell according to claim 11, **characterised in that** masks are designed to successively uncover each pixel family and to allow the progressive radiation thereof.

**14.** Display cell according to claim 11, **characterised in that** it does not include any partitioning, and **in that** the chiral doping agents according to claim 1 have a polymerisable or co-polymerisable end chain.

**15.** Display cell according to claim 11, **characterised in that** it includes partitions for each zone or for each pixel, and **in that** the chiral doping agents according to claim 1 may not include a polymerisable or co-polymerisable end chain.

## Patentansprüche

**1.** Chirale Dotierstoffe, die ermöglichen, eine Modifikation der Steighöhe einer cholesterischen Flüssigkristallzusammensetzung einzuführen, die eine chemische Struktur hat, die eine bifunktionale chirale Einheit enthält, wovon wenigstens eine der Funktionen ermöglicht, eine chemische Verbindung mit einer isomerisierbaren Gruppe herzustellen, wobei die Gruppe eventuell eine polymerisierbare oder copolymerisierbare Endkettenbildung besitzt, **dadurch gekennzeichnet, dass** sie der folgenden Formel I entsprechen:

$$\mathbf{A_1\text{-}Y_1\text{-}X\text{-}Y_2\text{-}A_2} \qquad \mathbf{(I)} \qquad (I)$$

worin

- X ein chirales Radikal repräsentiert, das aus einer bifunktionalisierten Verbindung abgeleitet ist,
- $Y_1$ und $Y_2$ gleich oder unterschiedlich sind und jeweils eine funktionale Verbindungseinheit repräsentieren, die gewählt ist aus -O-, -S-, -COO-, -OCO-,
- CON(R)- und -N(R)CO-, worin R einen Alkylrest repräsentiert,
- $A_1$ eine photoisomerisierbare Mesogen-Gruppe repräsentiert, die der folgenden Formel (II) entspricht:

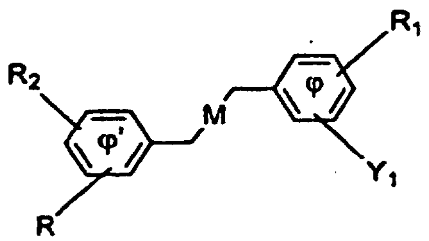

(II)

worin zwei Phenylreste φ und φ' durch ein photoisomerisierbares bivalentes Radikal -M-, das aus -N=N-, -N=CH-, -CH=N- und -CH=CH- gewählt ist, verbunden sind; wobei die Phenylreste jeweils einen oder mehrere Substituenten $R_1$, $R_2$ besitzen, die gleich oder verschieden sind und gewählt sind aus Wasserstoff, den Alkyl- oder Alkoxy-Radikalen von $C_1$ bis $C_5$, den Halogenen und den Cyano-, Nitro- oder Trifluormethyl-Radikalen; und R' Wasserstoff oder eine Gruppe repräsentiert, die der folgenden Formel (III) entspricht:

$$-Y_3-C_nH_{2n}-Z \qquad \textbf{(III)} \qquad \text{(III)}$$

worin $Y_3$ die gleichen Bedeutungen wie $Y_1$ und $Y_2$ hat oder Phenylen repräsentiert, n eine ganze Zahl im Bereich von 0 bis 12 ist und Z Wasserstoff oder einen polymerisierbaren Rest, der aus den Acryloyl- und Acryloyloxy-Resten gewählt ist, repräsentiert; und

- $A_2$ die gleiche Bedeutung wie die Gruppen der Formel (II) oder der Formel (III) hat.

**2.** Chirale Dotierstoffe nach Anspruch 1, **dadurch gekennzeichnet, dass** das bivalente Radikal -M- das Azo-Radikal -N=N- ist, das mit den Resten φ und φ' ein Azophenyl-Radikal bildet, und dass die Substituenten R und $Y_1$ oder $Y_2$ die gleichen Ortho-, Meta- oder Parapositionen an den Resten φ und φ' einnehmen.

**3.** Chirale Dotierstoffe nach Anspruch 1, **dadurch gekennzeichnet, dass** $A_1$ und $A_2$ jeweils einer photoisomerisierbaren Mesogen-Gruppe gemäß Formel II entsprechen.

**4.** Chirale Dotierstoffe nach Anspruch 1, **dadurch gekennzeichnet, dass** $A_2$ einer Gruppe der Formel III entspricht.

**5.** Chirale Dotierstoffe nach einem der Ansprüche 1 bis 4, **dadurch gekennzeichnet, dass** R eine Gruppe gemäß Formel III repräsentiert und dass Z immer ein polymerisierbares Radikal repräsentiert, das aus den Acryloyl- und Acryloyloxy-Radikalen gewählt ist.

**6.** Chirale Dotierstoffe nach Anspruch 1, **dadurch gekennzeichnet, dass** das chirale Radikal X aus einer zweiwertigen Säure oder aus einem chiralen Diol abgeleitet ist.

**7.** Chirale Dotierstoffe nach einem der Ansprüche 1 bis 6, **dadurch gekennzeichnet, dass** das chirale Radikal X den bifunktionalen Radikalen entspricht, die aus Dianhydroglucitol oder aus Dianhydromannitol abgeleitet sind.

**8.** Chiraler Dotierstoff nach Anspruch 7, **dadurch gekennzeichnet, dass** er der folgenden Verbindung entspricht:

**-2,5-Bis[4'-(6-acryloyloxyhexyloxy)-2',5'-methylazophenyl-4-carbonyloyl]-1,4;3,6-dianhydro-*D*-glucitol.**

**9.** Chiraler Dotierstoff nach Anspruch 7, **dadurch gekennzeichnet, dass** er der folgenden Formel entspricht:

**-2-[4'-(6-acryloyloxyhexyloxy)-2',5'-methylazophenyl-4-carbonyloyl]-5-[4-(6-acryloyloxyhexyloxy)benzoyl]-1,4;3,6-*D*-glucitol.**

**10.** Verfahren zum Modulieren der chiralen Dotierstoffe nach Anspruch 1, **dadurch gekennzeichnet, dass** die Modifikation der Induzierung der Steighöhe der chiralen Dotierstoffe durch Bestrahlung mit Ultraviolettstrahlung (UV), sichtbarer Strahlung (VIS) oder gemischter Strahlung (UV + VIS) erhalten wird, die auf die isomerisierbare Gruppe

einwirkt, wobei die Bestrahlungsdauer gesteuert wird, um eine bestimmte Farbe zu definieren, wenn die Dotierstoffe in eine cholesterische Flüssigkristallzusammensetzung eingebaut sind.

**11.** Anzeigezelle, die zwei transparente Substrate, die mit einer elektronischen Schaltung verbundene Elektroden unterstützen, und einen Einkapselungsrahmen, der einen lamellenförmigen Raum begrenzt, in den eine cholesterische Flüssigkristallzusammensetzung eingeführt ist, die wenigstens einen chiralen Dotierstoff nach Anspruch 1 enthält, umfasst, **dadurch gekennzeichnet, dass** die Zelle einer Ultraviolettstrahlung (UV) oder einer sichtbaren Strahlung (VIS) unterworfen ist, die in Abhängigkeit von unterschiedlichen Zonen oder von unterschiedlichen Pixelfamilien durch die Verwendung von Masken und durch die Änderung der Bestrahlungsdauer moduliert wird.

**12.** Anzeigezelle nach Anspruch 11, **dadurch gekennzeichnet, dass** jede Pixelfamilie einer bestimmten Maske entspricht, die ermöglicht, die Bestrahlungsdauer jeder Familie einzustellen.

**13.** Anzeigezelle nach Anspruch 11, **dadurch gekennzeichnet, dass** die Masken so beschaffen sind, dass sie nacheinander jede Pixelfamilie abdecken und ihre allmähliche Bestrahlung ermöglichen.

**14.** Anzeigezelle nach Anspruch 11, **dadurch gekennzeichnet, dass** sie keinerlei Unterteilung besitzt und dass die chiralen Dotierstoffe nach Anspruch 1 eine polymerisierbare oder copolymerisierbare Endkettenbildung besitzen.

**15.** Anzeigezelle nach Anspruch 11, **dadurch gekennzeichnet, dass** sie für jede Zone oder für jedes Pixel Unterteilungen enthält und dass die chiralen Dotierstoffe nach Anspruch 1 keine polymerisierbare oder copolymerisierbare Endkettenbildung zu besitzen brauchen.

Figure 1

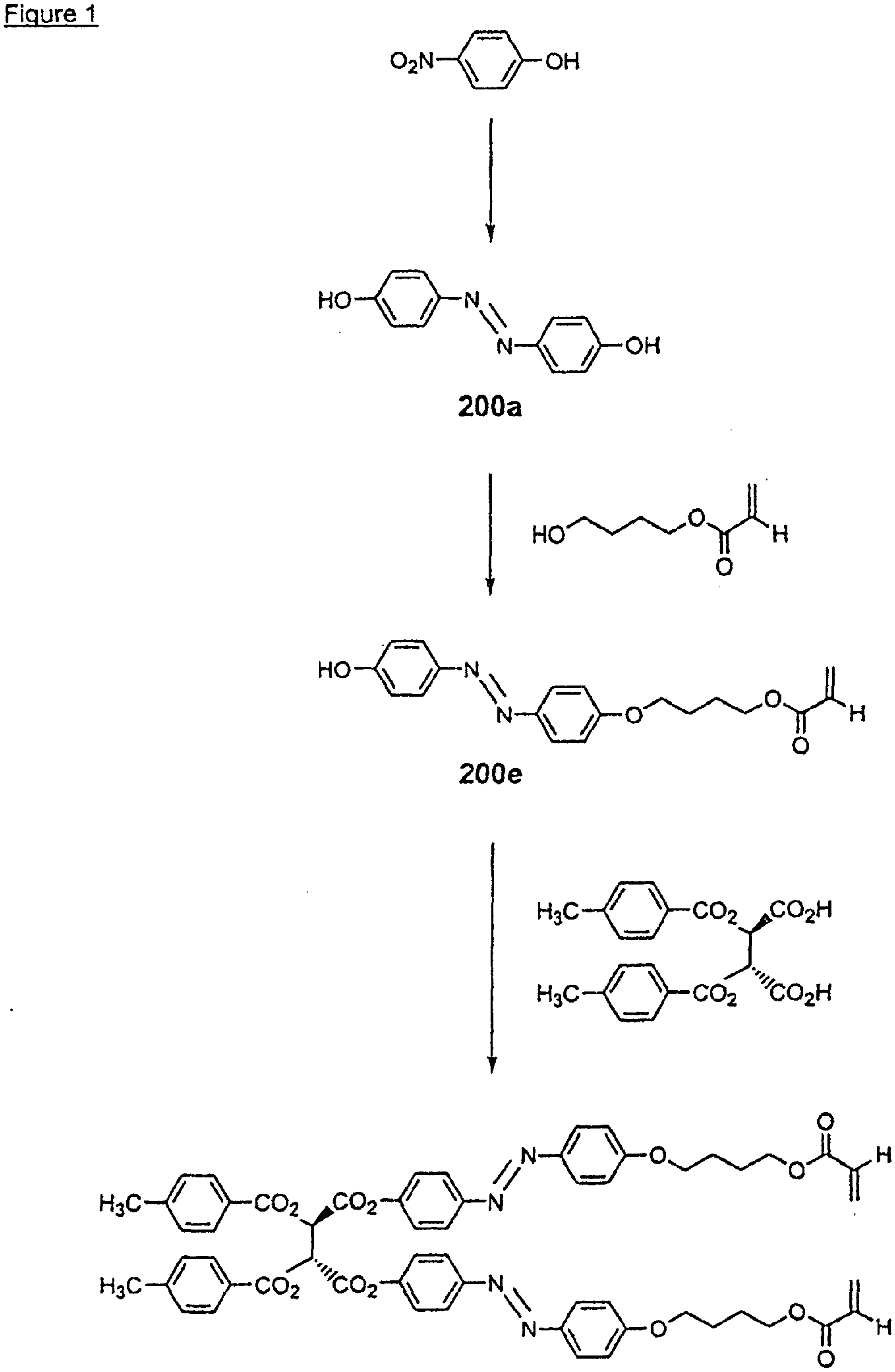

200a

200e

201e

Figure 2

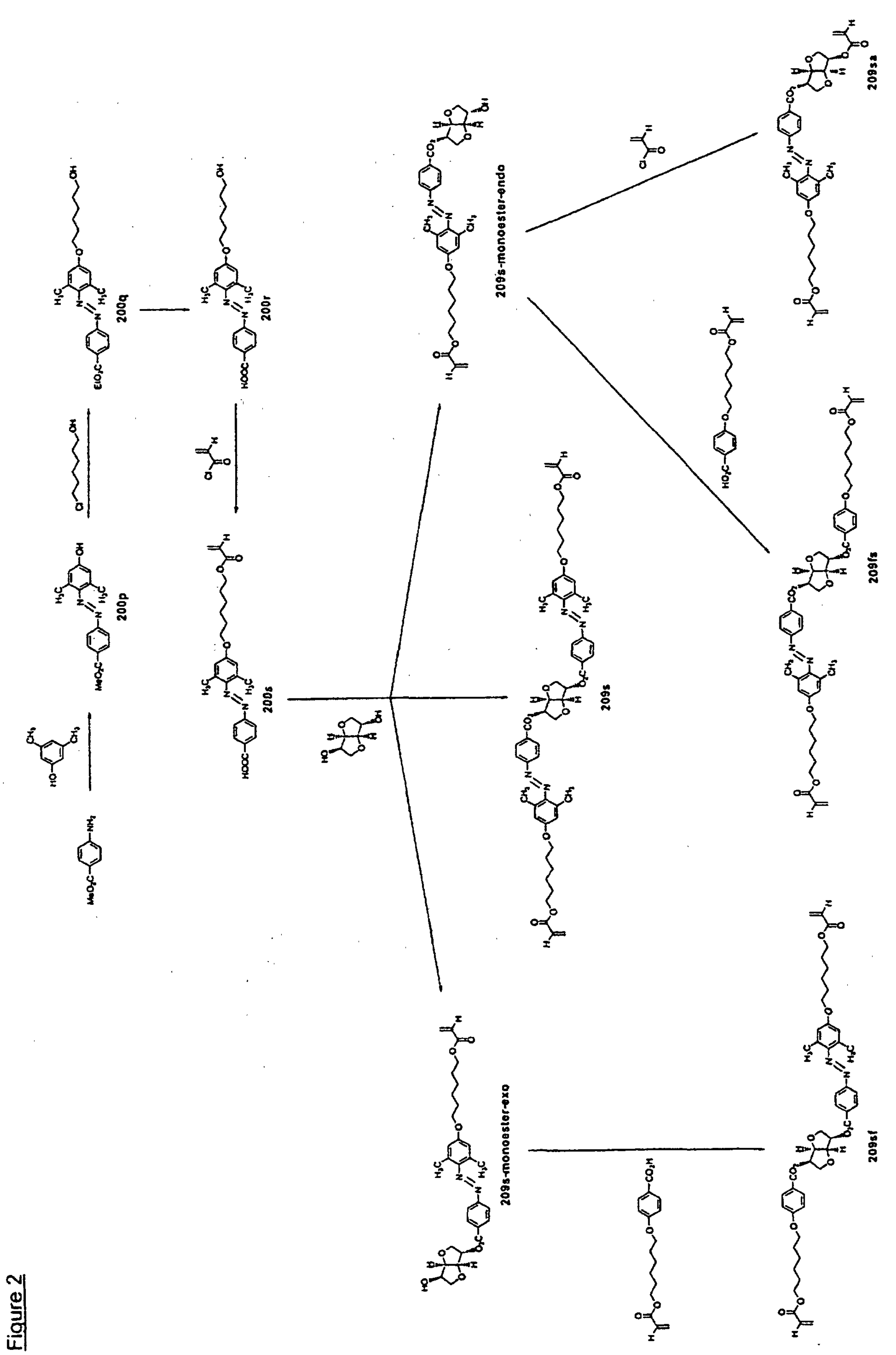

200p

200q

200r

200s

209s

209s-monoester-endo

209s-monoester-exo

209sa

209fs

209sf

Figure 3

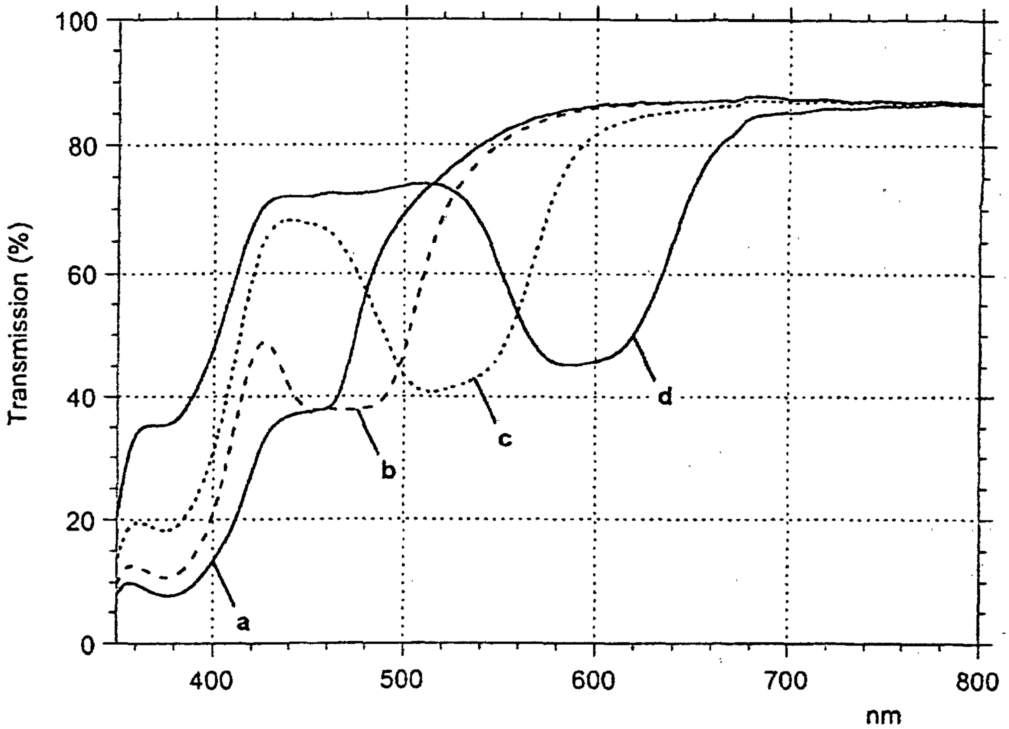


Figure 4

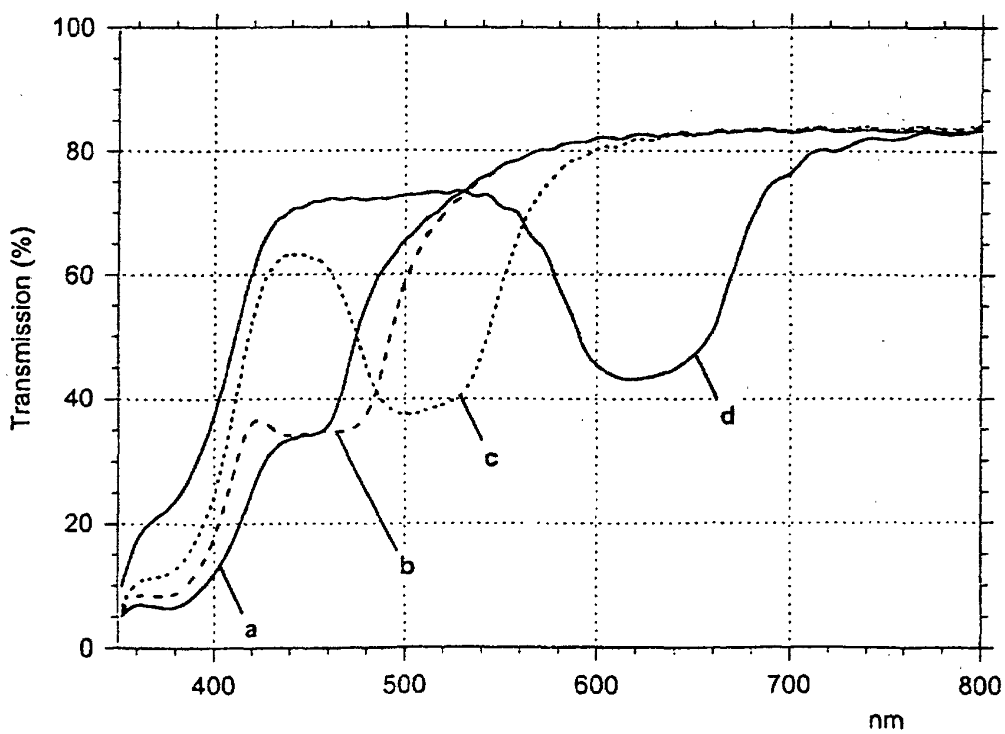

Figure 5

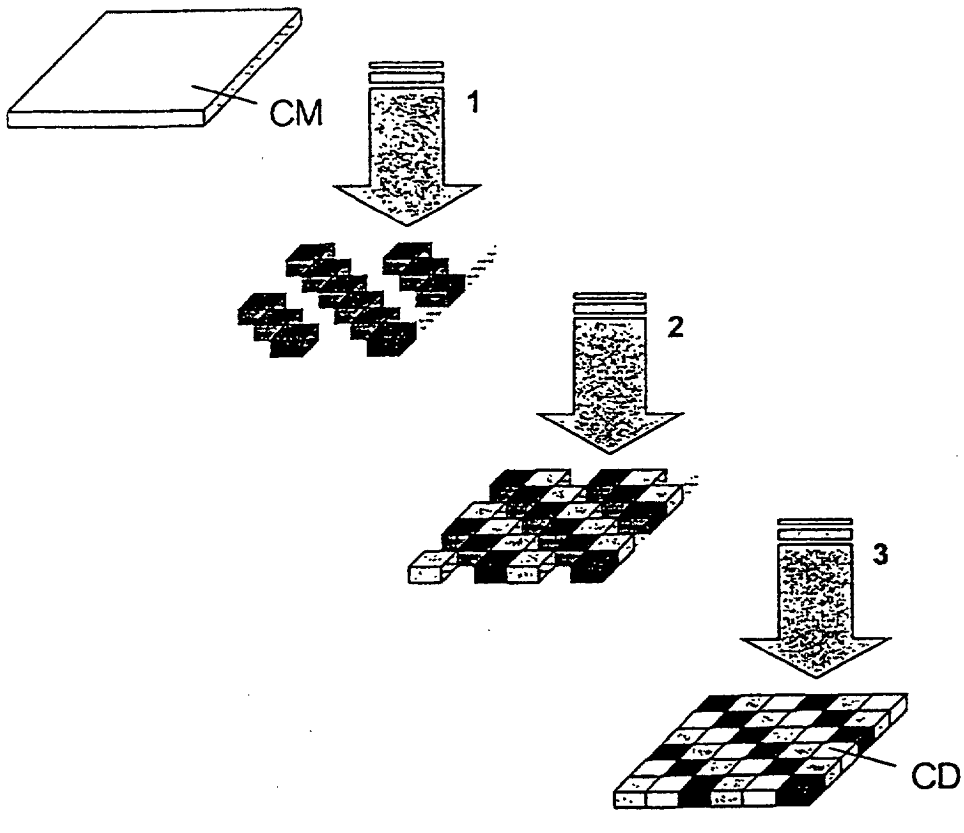

Figure 6

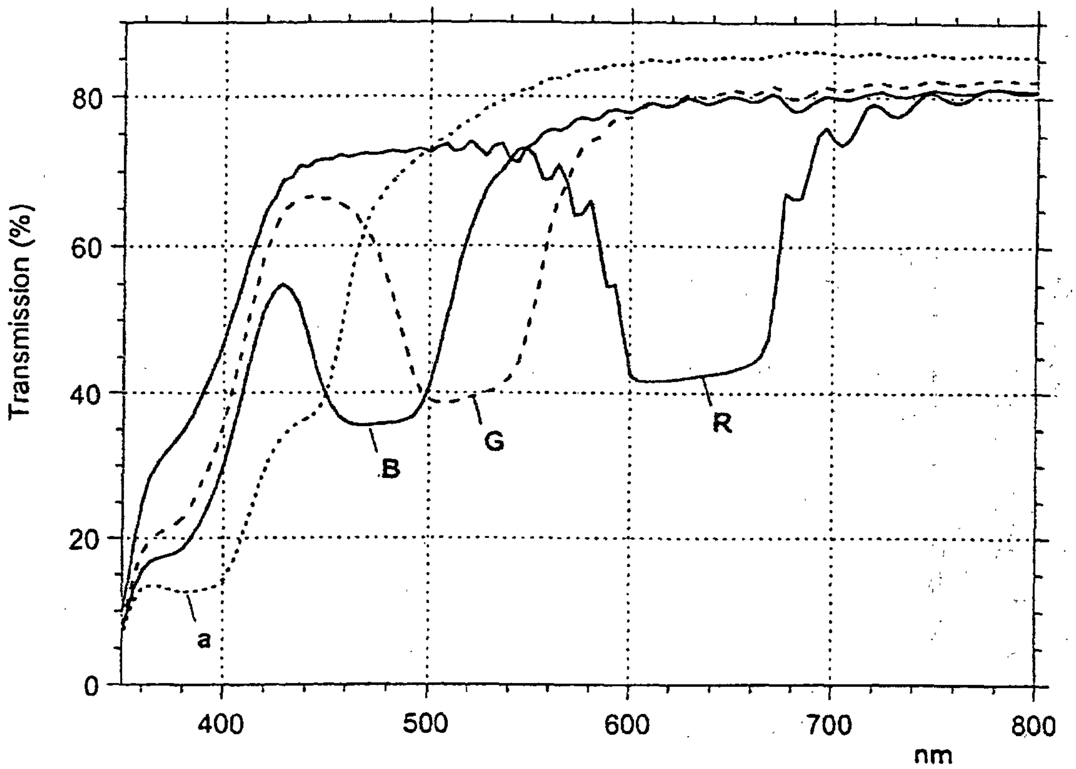


Figure 7